Europäisches Patentamt

European Patent Office

Office européen des brevets

(19)

(11) **EP 0 772 772 B1**

(12) **EUROPEAN PATENT SPECIFICATION**

(45) Date of publication and mention
of the grant of the patent:
**04.11.1998 Bulletin 1998/45**

(51) Int Cl.$^6$: **G01N 33/18**, G01N 15/06

(21) Application number: **95928459.7**

(22) Date of filing: **21.07.1995**

(86) International application number:
**PCT/EP95/02937**

(87) International publication number:
**WO 96/03645 (08.02.1996 Gazette 1996/07)**

(54) **A METHOD AND APPARATUS FOR DETERMINING THE CONCENTRATION OF A FIRST FLUID WHICH IS FINELY DIVIDED IN A SECOND FLUID**

VERFAHREN UND VORRICHTUNG ZUR BESTIMMUNG DER KONZENTRATION EINER ERSTEN FLÜSSIGKEIT, DIE IN EINER ZWEITEN FLÜSSIGKEIT FEIN VERTEILT IST

PROCEDE ET APPAREIL PERMETTANT DE DETERMINER LA CONCENTRATION D'UN PREMIER FLUIDE QUI EST FINEMENT DISPERSE DANS UN DEUXIEME FLUIDE

(84) Designated Contracting States:
**BE DE DK FR GB NL SE**

(30) Priority: **22.07.1994 EP 94202147**

(43) Date of publication of application:
**14.05.1997 Bulletin 1997/20**

(73) Proprietor: **SHELL INTERNATIONALE RESEARCH MAATSCHAPPIJ B.V.**
**2596 HR Den Haag (NL)**

(72) Inventors:
  • **VAN DEELEN, Christiaan, Charles, Johannes**
**NL-2288 GD Rijswijk (NL)**
  • **HOLWEG, Philippus, Antonius, Maria**
**NL-2288 GD Rijswijk (NL)**
  • **HUIZER, Willem**
**NL-2288 GD Rijswijk (NL)**

(56) References cited:
**EP-A- 0 382 882**       **US-A- 4 102 177**
**US-A- 4 809 543**

  • **PATENT ABSTRACTS OF JAPAN vol. 004 no. 722 (E-77) ,22 April 1977 & JP,A,52 050293 (YAMATAKE HONEYWELL) 21 October 1975,**
  • **PATENT ABSTRACTS OF JAPAN vol. 9 no. 24 (P-331) [1737] ,31 January 1985 & JP,A,59 170747 (TOKYO KEIKI K.K.) 27 September 1984,**
  • **PATENT ABSTRACTS OF JAPAN vol. 001 no. 063 (E-025) ,20 June 1977 & JP,A,52 004290 (TOEI DENSHI KOGYO K.K.) 13 January 1976,**

## Description

The present invention relates to a method for determining the concentration of a first fluid which is finely divided in a second fluid. Generally, a system wherein a first fluid is finely divided in a second fluid is defined a dispersed system, i.e. the first fluid (dispersed phase) is wandering about the second fluid (dispersing medium) in a finely divided form.

As known to those skilled in the art, dispersed systems can be sub-divided as follows: dependent on the diameter of the dispersed phase particles, a dispersed system can be a solution (homogeneous mixture in which no settling occurs and in which solute particles are at the molecular or ionic state of subdivision); a colloidal system (an intermediate kind of mixture in which the solute-like particles are suspended in the solvent-like phase and in which the particles of the dispersed phase are small enough that settling is negligible and large enough to make the mixture appear cloudy); or a suspension (a clearly heterogeneous mixture in which solute-like particles immediately settle out after mixing with a solvent-like phase).

In particular, the invention relates to detecting and measuring the concentration of a hydrocarbon (oil) emulsified in a water stream, wherein oil may be present in water in the form of droplets having a certain size and distribution.

However, it will be appreciated that the invention is not restricted to the measurement of the concentration of oil in water, but can suitably be applied to determine the concentration of any first fluid which is finely divided in a second fluid, provided that the particle size distribution of the first fluid within the second fluid can be changed. Examples are liquid in liquid, gas in liquid or liquid in gas.

At present, the continuous on-line measurement of oil-in-water concentration is becoming increasingly important in order to meet future effluent quality standards.

Concern over the impact of industrial activities on the environment, often heightened by public concern and legislative requirements, has led to a re-evaluation of the wastes and discharges which routinely occur.

Historically, produced water has been disposed of to the environment after treatment, either by release to surface waters or by re-injection into suitable aquifiers or the production formations themselves. Strict quality levels are imposed by statutory authorities and these vary from maximum oil concentrations of 5 mg/l for discharge to fresh water systems, to oil concentrations of 40 mg/l for water streams discharged to the open sea. Current trends will result in reduction of these levels within the foreseeable future. New limits of 30 mg/l have been proposed. This will call for the on-line measurement of the discharge streams, and closer/improved control of the water treatment facilities, if the new standards are to be met.

A variety of instruments is known for measuring oil present in water streams, originating from oil production operations.

Water streams from oil production operations do not only contain dispersed hydrocarbons in the form of droplets, but also contain sand, scale and corrosion particles.

US-A-4 102 177 discloses a method of determining the concentration of oil in water. wherein detector signals are obtained from two emulsification degrees of the oil/water mixture. The detector signals represent intensities It(x) and It'(x) of transmitted light, wherein x is the concentration of oil in water and the two emulsification degrees are represented by proportional coefficients $\alpha$ and $\alpha'$. The magnitude of x is determined using the relationship

$$x = (1/\Delta\alpha) \log (It'(x)/It(x))$$

in which $\Delta\alpha = \alpha - \alpha'$

Thus, in order to determine the oil concentration x it is required to determine the degrees of emulsification $\alpha$ and $\alpha'$ beforehand.

An important limitation of commercially available instruments for on-line monitoring of oil in produced water is the poor ability to discriminate between oil droplets and suspended particles. The operation of on-line monitors is influenced by these particles, which makes these monitors unreliable. This poor discrimination ability arises from the fact that for many of the applied measurement techniques the particles and the droplets give a rather similar measurement signal.

Clearly, a measurement technique, where only the droplets produce a measurement signal, while particles do not contribute to the measurement signal, would overcome the above-mentioned problem.

As already indicated in the foregoing, measuring the quality of produced water is important in reducing the environmental impact of discharges from oil production operations and for determining whether the water quality meets the requirements set by legislation. Currently, at regular intervals produced water samples are collected manually, transported to a laboratory, and analysed. A frequently used analysis technique is infra-red spectrophotometry. On-line monitoring will allow unattended and continuous measurement of produced water quality. Cost savings can be achieved as a result of platform demanding.

It is an object of the invention to provide a reliable method and apparatus for on-line determining the concentration of a first fluid which is finely divided in a second fluid, based upon varying the particle size distribution of the said first fluid in the second fluid enabling the accurate detection and measurement of (low) concentration of said first fluid present in the second fluid, wherein the method and apparatus are sensitive to changes in particle size distribution of said first fluid and wherein finely divided solids or any other non-first fluid particles which may be present in the second fluid do not influence the first fluid concentration measurement.

The invention therefore provides a method for determining the concentration of a first fluid which is finely divided in a second fluid, comprising the steps of:

a) producing a sample of fluid to be analysed having a first well defined particle size distribution of the first fluid within the second fluid;

b) supplying the said sample to a detector, which is sensitive to changes in particle size distribution of the first fluid within the second fluid, and measuring at least a first detector signal of the said sample flowing through the said detector, said first detector signal corresponding to the said first well defined particle size distribution;

c) introducing at least one change in the said particle size distribution of the first fluid within the second fluid,

d) measuring at least a second detector signal of the said sample flowing through the said detector, said second detector signal corresponding to the second specific particle size distribution of the first fluid within the second fluid, and

e) deriving from the detector signal change, thus obtained, information on the concentration of the first fluid finely divided in the said second fluid, characterized in that

the applied detector technique is selected from ultrasound scattering, invisible light or visible light scattering, invisible light or visible light blocking, and turbidity measurements, wherein the calculation of the said first fluid concentration is based upon the difference in two neasured intensity signals, and wherein the relative reduction of the light intensity R is determined using:

$$R = k \cdot \frac{i_{low} - i_{high}}{i_{low} + i_{high}}$$

wherein k is a constant, e.g. 2, and $i_{low}$ and $i_{high}$ are the intensity signals measured at relatively low and relatively high rotation speeds respectively.

The invention further provides an apparatus for determining the concentration of a first fluid finely divided in a second fluid, comprising means for producing a sample of fluid to be analysed having a well defined particle size distribution of the first fluid within the second fluid; means for supplying the said sample to a detector, which is sensitive to changes in particle size distribution of the first fluid within the second fluid;

means for introducing at least one change in the said particle size distribution of the first fluid within the second fluid; means for measuring at least two detector signals of the said sample flowing through the said detector, each detector signal corresponding to the said specific particle size distributions of the first fluid within the second fluid;

means for deriving from the detector signal change, thus obtained, information on the concentration of the first fluid finely divided in the said second fluid.

Advantageously, in step c) of the method of the invention, more than one change in particle size distribution of the first fluid within the second fluid are effected to increase the sensitivity of the measurement.

The invention will now be described in more detail by way of example by reference to the accompanying drawings, in which:

fig. 1 represents schematically the principle of the method of the invention; and

fig. 2 represents schematically an advantageous embodiment of the present invention.

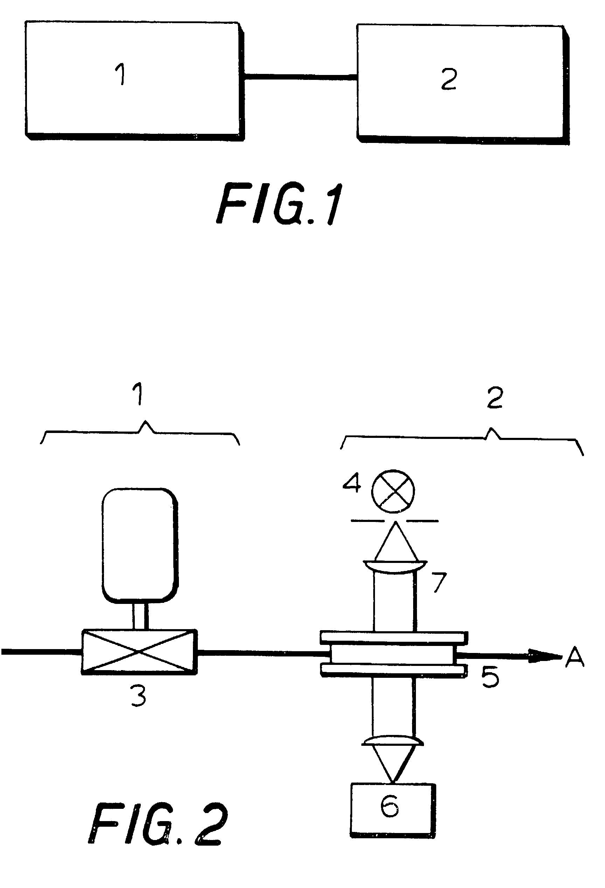

Referring to fig. 1 a block scheme of the method of the invention to determine the first (dispersed) fluid concentration in a second fluid using controlled changes in particle size distribution of the first fluid is shown.

Block 1 represents a sample conditioning system and block 2 represents an analysing system comprising a detector sensitive to changes in particle size distribution. As already indicated in the foregoing, the invention is based on the fact that the properties of a dispersing medium change when the size distribution of the particles forming the dispersed fluid is changed.

The principle of the invention is based upon an extension of the conditioning step for producing a sample of fluid to be analysed having a well defined particle size distribution of the first fluid within the second fluid.

The sample conditioning steps should only affect the particle size distribution of the first fluid and should not affect the size distribution of finely divided solids or any other non-first fluid particles which may also be dispersed in the second fluid.

Thus, a signal change measured by the detection system will only be caused by the change in properties of the first (dispersed) fluid. As a result, this signal change can be directly related to the concentration of the first (dispersed) fluid. Such a sample conditioning step can be carried out by a controlled change in particle size distribution of the first fluid. Advantageously, mixers with different mixing intensities can be used to condition a sample of fluid to be analysed. With appropriate known parameters such as mixer-rotor-diameter and the rotation speeds, the particle size distribution can be determined, as is known to those skilled in the art. The fluid sample to be analysed which is to be supplied to the detector, may or may not have a known particle size distribution of the first fluid within the second fluid. It will be appreciated by those skilled in the art that in an earlier process stage the particle size distribution may already have been determined or is already known dependent on appropriate process conditions, e.g. the use of a hydrocyclone. If the particle size distribution is not known, the sample should be conditioned first e.g. by mixing to obtain a well defined particle size distribution of the first fluid within the second fluid.

The dispersed fluid concentration can be determined using the following steps:

1. Introducing at least a change in the size distribution of the particles forming the dispersed fluid.
2. Measuring at least two detector signals of the analysing system.
3. Calculation of the dispersed fluid concentration from the measured detector signal change.

Various implementations of the invention are possible. For example, suitable particle size distributions can be produced by a mechanical shear mixer, an ultrasonic mixer or a high pressure orifice homogeniser. Further, the analysing system can apply e.g. an optical technique, an acoustical technique or a droplet counting technique.

As will be appreciated by those skilled in the art the detection principles of the analysing system may vary from ultrasound scattering, (visible) light scattering, (visible) light blocking to turbidity measurements.

Referring to Fig. 2 an oil-in-water-monitor as an advantageous embodiment of the invention is schematically shown.

In case of an oil-in-water system, light scattered by oil droplets is sensitive to the droplet diameter. Therefore, light scattering is a suitable technique to detect the change in droplet size distribution.

The apparatus comprises a sample conditioning system 1 and an analysing system 2. The sample conditioning system 1 comprises a homogenising/mixing device 3, which can be controlled in any way suitable for the purpose to produce different droplet size distributions of the oil in the water. A controlled change is possible, because oil droplets are relatively soft, while the other particles are relatively hard. The analysing system 2 comprises a light source 4, a sample cell 5, a detector 6 and suitable optics 7. The arrow A represents fluid flow.

In an advantageous embodiment of the present invention the detection principle is light scattering at a single wavelength.

The sample of produced water is fed through a suitable high speed mixer (e.g. rotor diameter 50 mm), which is operated in an alternating way at a relatively low rotation speed (e.g. 4500-5500 rpm) and a relatively high rotation speed (e.g. 6300-7300 rpm). Typically the mixer speed is changed every minute. Subsequently, the conditioned water is fed through the sample cell 5. A beam of near-infra-red light at a wavelength of 1.3 μm is directed in any suitable manner through the sample cell 5. The light which is transmitted and scattered under angles of up to 10° is collected on the detector. The near-infra-red beam is created by passing the light from any suitable light source such as a tungsten filament lamp through a narrow band interference filter.

As already indicated in the foregoing the dispersed fluid (oil) concentration can be determined using the following steps:

1. Operation of the homogeniser/mixer to produce a first droplet size distribution and detection of a signal corresponding to the light scattered by these droplets. It should be noted that in this step operation of the homogeniser/mixer is not required when the first droplet size distribution is already known.
2. Operation of the homogeniser/mixer to produce a second, different, droplet size distribution and detection of a signal corresponding to the light scattered by these droplets.
3. Calculation of the dispersed fluid (oil) concentration from the change in detector signals from (1) and (2).

Non-oil particles present in the produced water do not influence the concentration measurement, because these particles only give background signals, which do not contribute to the observed signal differences.

It is assumed that the dispersed fluid (oil) concentration in steps (1) and (2) is the same. The characteristic detector signal change corresponding to the different droplet size distributions as a function of dispersed fluid (oil) concentration can be obtained from a calculation using scattering theory (known as such) and droplet size distribution information or simply from a calibration measurement.

Advantageously, measurements can be carried out with the oil-in-water monitor based upon multiple-wavelength described in European patent application No. 93306972 filed September 3, 1993.

The use of more than one (e.g. eight) wavelength would have the advantage of an improved accuracy in oil concentration, would allow to correct for temperature effects and would allow a correction for possible changes in mixing characteristics.

The calculation of the dispersed fluid (oil) concentration from the change in detector signals is as follows:

From the signals measured at the relatively low rotation speed cycle $i_{low}$ and relatively high rotation speed cycles $i_{high}$, only the difference in signal is analysed. Specifically, the small relative reduction of the light intensity R is determined using

$$R = k \cdot \frac{i_{low} - i_{high}}{i_{low} + i_{high}} \qquad (1)$$

wherein k is a $i_{low} + i_{high}$ constant, e.g. 2

Next, the oil concentration c is calculated from the measured change in light reduction by

$$c = \alpha.R \qquad (2)$$

The factor $\alpha$ can be obtained experimentally by a calibration procedure, where the detector signal, in particular the relative light reduction R, has been measured with a well known concentration c of oil.

The factor a can also be obtained numerically. This requires a well characterized optical set-up and knowledge on the size distribution of the droplets for the two mixer speeds. With these parameters, it is possible to calculate the light scattering intensity measured by the detector and thus the factor $\alpha$ from light scattering theory.

By using Eq. 2 it is assumed that the light scattering intensity is proportional to the dispersed fluid (oil) concentration. This assumption is justified as long as the optical path through the sample cell is not too large.

The accuracy of the different mixer speed method can be further improved by using larger differences in mixer speed or by using different optical arrangements.

The different mixer speed method is an advantageous technique for determining the oil concentration in the presence of unknown types of particles. The method is attractive for determining the oil concentration in produced water, where the type of particles is generally unknown and can change as a function of time and location.

Various modifications of the present invention will become apparent to those skilled in the art from the foregoing description. Such modifications are intended to fall within the scope of the appended claims.

## Claims

1. A method for determining the concentration of a first fluid which is finely divided in a second fluid, comprising the steps of:

   a) producing a sample of fluid to be analysed having a first well defined particle size distribution of the first fluid within the second fluid,
   b) supplying said sample to a detector, which is sensitive to changes in particle size distribution of the first fluid within the second fluid; and measuring at least a first detector signal of the said sample flowing through the said detector, said first detector signal corresponding to the said first well defined particle size distribution;
   c) introducing at least one change in the said particle size distribution of the first fluid within the second fluid,
   d) measuring at least a second detector signal of said sample flowing through the said detector, said second detector signal corresponding to the second specific particle size distribution of the first fluid within the second fluid, and

   e) deriving from the detector signal change, thus obtained, information on the concentration of the first fluid which is finely divided within the said second fluid, characterized in that the applied detector technique is selected from ultrasound scattering, invisible light or visible light scattering, invisible light or visible light blocking, and turbidity measurements, wherein the calculation of the said first fluid concentration is based upon the difference in two measured intensity signals, and wherein the relative reduction of the light intensity R is determined using

$$R = k \cdot \frac{i_{low} - i_{high}}{i_{low} + i_{high}}$$

   wherein k is a constant, e.g. 2 and $i_{low}$ and $i_{high}$ are the intensity signals measured at relatively low and relatively high rotation speeds respectively.

2. The method as claimed in claim 1, wherein in step c) two or more particle size distributions are effected.

3. The method as claimed in claim 1 or 2, wherein the particle size distribution in steps a) and/or c) are effected by a mixing or homogenising means, e.g. a mechanical shear mixer, a high pressure pump in combination with an orifice, or an ultrasonic device.

4. The method as claimed in claim 3, wherein different mixer intensities or rotation speeds are applied.

5. The method as claimed in any one of claims 1-4, wherein oil-in-water concentration is determined.

6. The method as claimed in claim 1, wherein the dispersed fluid concentration c is calculated from the measured change in light reduction by $c = \alpha.R$, wherein $\alpha$ is obtained by a calibration procedure.

## Patentansprüche

1. Verfahren zum Bestimmen der Konzentration eines ersten Fluides, das in einem zweiten Fluid fein verteilt ist, mit den Schritten:

   a) Erzeugen einer Probe des zu analysierenden Fluides mit einer ersten gut definierten Partikelgrößenverteilung des ersten Fluides im zweiten Fluid;

   b) Zuführen der Probe zu einem Detektor, welcher gegenüber Veränderungen in der Partikelgrößenverteilung des ersten Fluides im zweiten Fluid empfindlich ist; und Messen zumindest eines ersten Detektorsignales der durch den Detektor strömenden Probe, wobei das erste Detektorsignal der ersten gut definierten Partikelgrößenverteilung entspricht;

c) Herbeiführen zumindest einer Veränderung in der Partikelgrößenverteilung des ersten Fluides im zweiten Fluid,

d) Messen zumindest eines zweiten Detektorsignales der durch den Detektor strömenden Probe, wobei das zweite Detektorsignal der zweiten speziellen Partikelgrößenverteilung des ersten Fluides im zweiten Fluid entspricht, und

e) Ableiten von Information über die Konzentration des im zweiten Fluid fein verteilten ersten Fluides aus der so erhaltenen Detektorsignaländerung, dadurch gekennzeichnet, daß das angewandte Detektorverfahren aus der Gruppe Ultraschallstreuung, Streuung von sichtbarem oder unsichtbarem Licht, Blockierung von sichtbarem oder unsichtbarem Licht, und Trübungsmessungen gewählt wird, wobei die Berechnung der ersten Fluidkonzentration auf der Differenz zweier gemessener Intensitätssignale basiert, und wobei die relative Reduktion der Lichtintensität R bestimmt wird mit Hilfe der Gleichung

$$R = k \cdot \frac{i_{low} - i_{high}}{i_{low} + i_{high}}$$

wobei k eine Konstante ist, z.B. 2, und $i_{low}$ und $i_{high}$ die bei verhältnismäßig geringen bzw. verhältnismäßig hohen Rotationsgeschwindigkeiten gemessenen Intensitätssignale sind.

2. Verfahren nach Anspruch 1, bei welchem im Schritt c) zwei oder mehr Partikelgrößenverteilungen herbeigeführt werden.

3. Verfahren nach Anspruch 1 oder 2, bei welchem die Partikelgrößenverteilung in den Schritten a) und/oder c) durch eine Misch- oder Homogenisiereinrichtung herbeigeführt wird, z.B. einen mechanischen Schermischer, eine Hochdruckpumpe in Verbindung mit einer kleinen Öffnung, oder eine Ultraschalleinrichtung.

4. Verfahren nach Anspruch 3, bei welchem verschiedene Mischerintensitäten oder Rotationsgeschwindigkeiten angewandt werden.

5. Verfahren nach einem der Ansprüche 1 bis 4, bei welchem die Konzentration von Öl in Wasser bestimmt wird.

6. Verfahren nach Anspruch 1, bei welchem die Konzentration c dses dispergierten Fluides aus der gemessenen Änderung der Lichtreduktion mit Hilfe von $c = \alpha.R$ berechnet wird, wobei $\alpha$ durch eine Kalibrierungsprozedur erhalten wird.

**Revendications**

1. Procédé pour la détermination de la concentration d'un premier fluide qui est finement divisé dans un deuxième fluide, lequel procédé comporte les étapes consistant à :

a) produire un échantillon de fluide à analyser, qui présente une première distribution granulométrique bien définie des particules du premier fluide dans le deuxième fluide;
b) introduire ledit échantillon dans un détecteur qui est sensible à des modifications de la distribution granulométrique des particules du premier fluide dans le deuxième fluide; et mesurer au moins un premier signal de détecteur sur ledit échantillon traversant ledit détecteur, ledit premier signal de détecteur correspondant à ladite première distribution granulométrique bien définie des particules;
c) introduire au moins une modification de ladite distribution granulométrique des particules du premier fluide dans le deuxième fluide;
d) mesurer au moins un deuxième signal de détecteur sur ledit échantillon traversant ledit détecteur; ledit deuxième signal de détecteur correspondant à ladite deuxième distribution granulométrique spécifique des particules du premier fluide dans le deuxième fluide, et
e) calculer à partir de la modification du signal de détecteur ainsi obtenue une information concernant la concentration du premier fluide finement divisé dans ledit deuxième fluide,

caractérisé en ce que la technique de détection utilisée est choisie parmi la dispersion d'ultrasons, la dispersion de lumière visible ou invisible, le blocage de lumière visible ou invisible et des mesures de turbidité, le calcul de ladite concentration en le premier fluide étant basé sur la différence entre deux signaux de mesure d'intensité, et dans lequel la réduction relative de l'intensité de la lumière R est déterminée à l'aide de :

$$R = k. (i_{bas} - i_{haut})/(i_{bas} + i_{haut})$$

dans laquelle k est une constante, par exemple 2, et $i_{bas}$ et $i_{haut}$ sont les signaux d'intensité mesurés respectivement à une vitesse de rotation relativement faible et à une vitesse de rotation relativement élevée.

2. Procédé selon la revendication 1, dans lequel, dans l'étape c), on réalise deux ou plusieurs distributions granulométriques des particules.

3. Procédé selon les revendications 1 ou 2, dans lequel la distribution granulométrique des particules dans les étapes a) et/ou c) est réalisée par un moyen de mélange ou d'homogénéisation, par exemple un mélangeur mécanique à cisaillement, une pompe à haute pression combinée à un orifice ou un dispositif à ultrasons.

4. Procédé selon la revendication 3, dans lequel on utilise différentes intensités de mélange ou différentes vitesses de rotation.

5. Procédé selon l'une quelconque des revendications 1 à 4, dans lequel on détermine la concentration de l'huile-dans-l'eau.

6. Procédé selon la revendication 1, dans lequel la concentration en fluide dispersé est calculée à partir de la modification mesurée de la réduction de la lumière, par $c = \alpha.R$, $\alpha$ étant obtenu par une opération d'étalonnage.

## FIG.1

## FIG.2